# EUROPEAN PATENT APPLICATION

(11) **EP 4 052 717 A1**
(43) Date of publication of application: **07.09.2022**
(21) Application number: 22161046.2
(22) Date of filing: 12.03.2013
(51) Int. Cl.: A61K 35/18, C12N 5/078, A61K 39/12, A61K 39/00, A61K 39/02

(54) **MODIFIED ERYTHROCYTE PRECURSOR CELLS AND USES THEREOF**

(30) Priority: 13.03.2012 US 201261610355 P; 15.03.2012 US 201261611402 P
(62) Divisional of application: 17183779.2
(71) Applicant: Celularity Inc., Florham Park, NJ 07932 (US)
(72) Inventor: EDINGER, James, Belford (US); ABBOT, Stewart, San Diego (US); LIANG, Bitao, Closter (US)
(74) Representative: Mewburn Ellis LLP

(57) **Abstract**

Provided herein are modified erythrocyte precursor cells, erythrocytes generated from modified erythrocyte precursor cells, and uses thereof.

## Description

This application claims priority benefit of U.S. Provisional Patent Application No. 61/610,355, filed March 13, 2012, and U.S. Provisional Patent Application No. 61/611,402, filed March 15, 2012, each of which is herein incorporated by reference in its entirety.

### 1. INTRODUCTION

Provided herein are modified erythrocyte precursor cells, erythrocytes produced by said modified erythrocyte precursor cells, and uses thereof.

### 2. BACKGROUND

Despite the existence of therapies for treating diseases such as cancer, infection, and autoimmune diseases, as well as the significant amount of scientific and medical research dedicated annually to uncovering such therapies, such diseases remain highly prevalent. As such, there remains a need for new and effective therapeutics for treatment of cancer, infection, and autoimmune diseases, as well as other diseases.

### 3. SUMMARY

In one aspect, provided herein are methods for modifying erythrocyte precursor cells, such that the modified erythrocyte precursor cells express one or more nucleic acids (e.g., RNA, DNA, or synthetic polynucleotide) that encode one or more polypeptides that are not normally expressed by said erythrocyte precursor cells. In specific embodiments, the modified erythrocyte precursor cells generated in accordance with the methods described herein produce erythrocytes, wherein the polypeptides that are not normally expressed by the erythrocyte precursor cells are present on or in the erythrocytes produced by the modified erythrocyte precursor cells. Also provided herein are modified erythrocytes, and populations thereof, produced by the modified erythrocyte precursor cells described herein. Such modified erythrocytes comprise proteins that are not normally found on or in erythrocytes.

In a specific embodiment, provided herein are modified erythrocytes that comprise a polypeptide that is not normally found on or in erythrocytes, wherein said polypeptide performs at least one physiological function that is lacking in a host to which the erythrocyte is administered.

In another specific embodiment, provided herein are modified erythrocytes that comprise a polypeptide that is not normally found on or in erythrocytes, wherein said polypeptide provides a therapeutic benefit to a subject to whom the erythrocytes are administered, i.e., the polypeptide possesses one or more activities that confer a therapeutic benefit to said subject.

In another specific embodiment, provided herein are modified erythrocytes that comprise a polypeptide that is not normally found on or in erythrocytes, wherein said polypeptide is an antigen, and wherein the presence of said antigen in or on said erythrocytes is sufficient to cause an immune response against said antigen in a subject to whom the erythrocytes are administered. In particular embodiments, the antigen is present on the erythrocytes. In certain embodiments, the antigen is a tumor antigen, i.e., a protein or antigenic fragment thereof known to be present on or in tumor cells of at least one type of cancer, e.g., a tumor-specific antigen. In certain embodiments, the antigen is a viral antigen, i.e., a protein or antigenic fragment thereof from a virus. In certain embodiments, the antigen is a bacterial antigen, i.e., a protein or antigenic fragment thereof from a bacterium. In certain embodiments, the antigen is one that is capable of inducing self-tolerance to the antigen in a subject to whom the antigen is administered. For example, the antigen is one that, while native to the subject, is normally recognized by the immune system of the subject as foreign, e.g., the antigen is one associated with an autoimmune disease.

In certain embodiments, the modified erythrocyte precursor cells described herein may be modified to express a nucleic acid (e.g., an RNA, DNA, or a synthetic polynucleotide) that encodes a polypeptide capable of modulating an immune function in a subject, i.e., the polypeptide is an immunomodulatory polypeptide, wherein said immunomodulatory polypeptide is present on or in erythrocytes produced by said modified erythrocyte precursor cells. In certain embodiments, modified erythrocyte precursor cells described herein may be modified to express a nucleic acid (e.g., an RNA, DNA, or a synthetic polynucleotide) that encodes a polypeptide that confers one or more properties to said modified erythrocyte precursor cells that is not normally associated with the cells, e.g., the polypeptide makes the modified erythrocyte precursor cells more susceptible to phagocytosis or possess a longer life span (e.g., can undergo more population doublings) than unmodified erythrocyte precursor cells.

In certain embodiments, the modified erythrocyte precursor cells, and/or modified erythrocytes produced by such cells comprise an opsonin, i.e., the modified erythrocyte precursor cells and/or modified erythrocytes comprise (e.g., are engineered to express or have bound to them) a molecule that results in immune targeting of the modified erythrocyte precursor cells and/or modified erythrocytes. In specific embodiments, the opsonin targets the modified erythrocyte precursor cells and/or modified erythrocytes for phagocytosis, e.g., phagocytosis by macrophages. For example, a modified erythrocyte precursor cell provided herein can express a nucleic acid (e.g., an RNA, DNA, or a synthetic polynucleotide), e.g., an antigen, and further comprise an opsonin. In a specific embodiment, provided herein are modified erythrocytes that comprise a nucleic acid (e.g., an RNA, DNA, or a synthetic polynucleotide) and an opsonin. Exemplary opsonins include, without limitation, immunoglobulins (e.g., IgG, antibodies), molecules that activate the complement system (e.g., C3b, iC3b, and C1q), molecules that inactivate complement system inhibitors such as DAF and CD59 (e.g., molecules such as streptavidin that can induce clustering of complement system inhibitors), and molecules that activate natural killer cells (e.g., IL-12, IL-15, IL-18, IL-2, and CCL5).

In another aspect, provided herein are compositions, e.g., pharmaceutical compositions, comprising the modified erythrocyte precursor cells described herein. Also provided herein are compositions, e.g., pharmaceutical compositions, comprising modified erythrocytes produced by the modified erythrocyte precursor cells described herein.

In another aspect, provided herein are methods of treating diseases and disorders in subjects in need thereof, comprising administering to said subjects erythrocytes produced by the modified erythrocyte precursor cells described herein.

In a specific embodiment, provided herein are methods of treating cancer in a subject in need thereof, comprising administering to said subject modified erythrocytes, or a composition comprising modified erythrocytes, wherein said modified erythrocytes comprise one or more polypeptides known to be efficacious in the treatment of cancer (e.g., cytokines).

In another specific embodiment, provided herein are methods of treating cancer in a subject in need thereof, comprising administering to said subject modified erythrocytes, or a composition comprising modified erythrocytes, wherein said modified erythrocytes comprise one or more tumor antigens. In accordance with such methods, the administration of said modified erythrocytes is sufficient to induce an immune response against cancer(s) that express the one or more tumor antigens.

In another specific embodiment, provided herein are methods of treating a viral disease or infection in a subject in need thereof, comprising administering to said subject modified erythrocytes, or a composition comprising modified erythrocytes, wherein said modified erythrocytes comprise one or more viral antigens. In accordance with such methods, the administration of said modified erythrocytes is sufficient to induce an immune response against viruses that express the one or more tumor antigens.

In another specific embodiment, provided herein are methods of treating a bacterial disease or infection in a subject in need thereof, comprising administering to said subject modified erythrocytes, or a composition comprising modified erythrocytes, wherein said modified erythrocytes comprise one or more bacterial antigens. In accordance with such methods, the administration of said modified erythrocytes is sufficient to induce an immune response against bacteria that express the one or more tumor antigens.

In another specific embodiment, provided herein are methods of treating an allergy or autoimmune disease in a subject in need thereof, comprising administering to said subject modified erythrocytes, or a composition comprising modified erythrocytes, wherein said modified erythrocytes comprise one or more antigens capable of inducing self-tolerance to the antigen in a subject to whom the antigen is administered. In accordance with such methods, the administration of said modified erythrocytes is sufficient to result in the recognition of said antigen by the subject's immune system as "self," and thus prevent an immune response from being generated against said antigen. In a specific embodiment, the modified erythrocytes may be administered in very large doses of antigen to induce self-tolerance. In other specific embodiments, the modified erythrocytes may be administered in small, repeated doses that are below the threshold required for stimulation of an immune response to induce self-tolerance.

In another aspect, provided herein are kits comprising the modified erythrocyte precursor cells and/or modified erythrocytes described herein, and or pharmaceutical compositions thereof.

### 4. DETAILED DESCRIPTION

### 4.1 ERYTHROCYTE PRECURSOR CELLS

Provided herein are modified erythrocyte precursor cells, modified erythrocytes produced by the modified erythrocyte precursor cells, and methods of making the modified erythrocyte precursor cells and erythrocytes.

Any type of cell known in the art that is capable of differentiating into an erythrocyte, i.e., any erythrocyte precursor cell, can be modified in accordance with the methods described herein to produce modified erythrocyte precursor cells. In certain embodiments, the erythrocyte precursor cells modified in accordance with the methods described herein are cells that are in the process of differentiating into an erythrocyte, i.e., the cells are of a type known to be exist during mammalian erythropoiesis. For example, the cells may be pluripotent hematopoietic stem cells (HSCs), multipotent myeloid progenitor cells, unipotent stem cells, pronormoblasts (preerythroblasts), early normoblasts (erythroblasts), intermediate normoblasts, late normoblasts, or reticulocytes. The modified erythrocyte precursor cells provided herein can be differentiated into modified erythrocytes using methods known in the art, using molecules known to promote erythropoiesis, e.g., SCF, Erythropoietin, IL-3, and/or GM-CSF.

In a specific embodiment, the erythrocyte precursor cells modified in accordance with the methods described herein express the cell surface antigen CD34, i.e., the erythrocyte precursor cells are CD34+. In another specific embodiment, the erythrocyte precursor cells modified in accordance with the methods described herein express the cell surface antigen CD133, i.e., the erythrocyte precursor cells are CD133+. In another specific embodiment, the erythrocyte precursor cells modified in accordance with the methods described herein are CD34+ and CD133+. In certain embodiments, the CD34+ cell is a CD34+ placental stem cell. See, e.g., U.S. patent application publication NO. 2003/0180269, the disclosure of which is hereby incorporated by reference in its entirety.

In certain embodiments, the erythrocyte precursor cells modified in accordance with the methods described herein produce an erythrocyte of a desired blood type/group, e.g., the cells may inherently produce or be engineered to produce erythrocytes corresponding to a specific ABO blood group or a specific Rh blood group.

In a specific embodiment, the erythrocyte precursor cells modified in accordance with the methods described herein produce an erythrocyte that belongs to the type O blood group. In another specific embodiment, the erythrocyte precursor cells modified in accordance with the methods described herein produce an erythrocyte that belongs to the type A blood group. In another specific embodiment, the erythrocyte precursor cells modified in accordance with the methods described herein produce an erythrocyte that belongs to the type B blood group. In another specific embodiment, the erythrocyte precursor cells modified in accordance with the methods described herein produce an erythrocyte that belongs to the type AB blood group. In another specific embodiment, the erythrocyte precursor cells modified in accordance with the methods described herein produce an erythrocyte that is Rh negative (Rh-) blood type. In another specific embodiment, the erythrocyte precursor cells modified in accordance with the methods described herein produce an erythrocyte that is Rh positive (Rh+) blood type. In another specific embodiment, the erythrocyte precursor cells modified in accordance with the methods described herein produce an erythrocyte that belongs to the type O blood group and is Rh-, i.e., the erythrocyte is of a type recognized in the art as a "universal donor."

In certain embodiments, the erythrocyte precursor cells modified in accordance with the methods described herein produce an erythrocyte that is blood type M, blood type N, blood type S, or blood type s; blood type P1; blood type Lua, blood type Lub, or blood type Lu(a); blood type K (Kell), k (cellano), Kpa, Kpb, K(a+), Kp(a-b-) or K-k-Kp(a-b-); blood type Le(a-b-), Le(a+b-) or Le(a-b+); blood type Fy a, Fy b or Fy(a-b-); and/or blood type Jk(a-b-), Jk(a+b-), Jk(a-b+) or Jk(a+b+).

In certain embodiments, when the erythrocyte precursor cells modified in accordance with the methods described herein are used to generate erythrocytes that are to be transplanted in a subject, the erythrocyte precursor cells can be syngeneic (i.e., genetically identical or closely related to the cells of the recipient subject), allogeneic (i.e., from a non-genetically identical member of the same species of the recipient subject) or xenogeneic (i.e., from a member of a different species than the recipient subject). Syngeneic cells include those that are autogeneic (i.e., from the recipient subject) and isogeneic (i.e., from a genetically identical but different subject, e.g., from an identical twin).

The erythrocyte precursor cells modified in accordance with the methods described herein may be obtained from any source known in the art, including donors (e.g., human donors), that are either living or cadaveric. For example, erythrocyte precursor cells may be harvested from a donor (including a potential recipient) using standard techniques known in the art. In certain embodiments, the erythrocyte precursor cells modified in accordance with the methods described herein are derived from a cell line, e.g., a hematopoietic stem cell line or another cell line capable of producing erythrocytes.

### 4.2 MODIFIED ERYTHROCYTE PRECURSOR CELLS

### 4.2.1 Methods of Modifying Erythrocyte Precursor Cells

Any method known in the art for engineering cells to express a nucleic acid that results in the production of a polypeptide of interest by the cell may be used to generate the modified erythrocyte precursor cells described herein.

In certain embodiments, erythrocyte precursor cells may be engineered to express a desired nucleic acid using virus-based vectors including, but not limited to, non-integrating replicating vectors, e.g., papilloma virus vectors, SV40 vectors, adenoviral vectors; integrating viral vectors, e.g., retrovirus vector or adeno-associated viral vectors; or replication-defective viral vectors. In a specific embodiment, the vector used to engineer erythrocyte precursor cells to express a desired nucleic acid is a non-integrating vector. Other methods of introducing DNA into erythrocyte precursor cells include the use of liposomes, electroporation, a particle gun, direct DNA injection, or the like.

In certain embodiments, erythrocyte precursor cells may be engineered to express a desired nucleic acid using zinc finger nucleases. In other embodiments, erythrocyte precursor cells may be engineered to express a desired nucleic acid using a transposon. See, e.g., Cannon et al., Curr Opin HIV AIDS, 2011, 6(1):74-9; and Sumiyoshi et al., Hum Gene Ther, 2009, 20(12):1607-26, the disclosures of which are hereby incorporated by reference in their entireties.

In certain embodiments, modified erythrocyte precursor cells can be generated by, e.g., transforming or transfecting erythrocyte precursor cells with DNA controlled by or in operative association with, one or more appropriate expression control elements, for example, promoter or enhancer sequences, transcription terminators, polyadenylation sites, or internal ribosomal entry sites. In certain embodiments, such a DNA may incorporate a selectable marker. Following the introduction of the foreign DNA, the modified erythrocyte precursor cells can be, e.g., grown in enriched media and then switched to selective media.

The DNA used to engineer the modified erythrocyte precursor cells can comprise any promoter known in the art to drive expression of a nucleotide sequence in mammalian cells, *e.g.,* human cells. For example, promoters include, but are not limited to, CMV promoter/enhancer, SV40 promoter, papillomavirus promoter, Epstein-Barr virus promoter, elastin gene promoter, and the like. In another specific embodiment, the promoter is one that is normally present in an erythrocyte precursor cell, e.g., an erythropoiesis-specific promoter that is expressed during one or more stages of erythropoiesis, such as the EKLF, GATA1, GATA2, HBb, HBg, LMO2, ZFPM1, and/or HOX4b promoter. In another specific embodiment, the promoter is regulatable so that the nucleotide sequence is expressed only when desired. Promoters can be either inducible (e.g., those associated with metallothionein and heat shock proteins) or constitutive. In another embodiment, the modified erythrocyte precursor cells may comprise a promoter that is tissue-specific or exhibits tissue specificity. Examples of such promoters include but are not limited to: myelin basic protein gene control region (Readhead et al., 1987, Cell 48:703) (oligodendrocyte cells); elastase I gene control region (Swit et al., 1984, Cell 38:639; Ornitz et al., 1986, Cold Spring Harbor Symp. Quant. Biol. 50:399; MacDonald, 1987, Hepatology 7:425) (pancreatic acinar cells); insulin gene control region (Hanahan, 1985, Nature 315:115) (pancreatic beta cells); myosin light chain-2 gene control region (Shani, 1985, Nature 314:283) (skeletal muscle).

In a specific embodiment, the erythrocyte precursor cells modified in accordance with the methods described herein are pluripotent hematopoietic stem cells (HSC). In another specific embodiment, the erythrocyte precursor cells modified in accordance with the methods described herein are multipotent myeloid progenitor cells. In another specific embodiment, the erythrocyte precursor cells modified in accordance with the methods described herein are unipotent stem cells. In another specific embodiment, the erythrocyte precursor cell modified in accordance with the methods described herein are pronormoblasts (preerythroblasts). In another specific embodiment, the erythrocyte precursor cells modified in accordance with the methods described herein are early normoblasts (erythroblasts). In another specific embodiment, the erythrocyte precursor cells modified in accordance with the methods described herein are intermediate normoblasts. In another specific embodiment, the erythrocyte precursor cells modified in accordance with the methods described herein are late normoblasts. In another specific embodiment, the erythrocyte precursor cells modified in accordance with the methods described herein are reticulocytes. In certain embodiments, the erythrocyte precursor cells modified in accordance with the methods described herein are CD34+. In certain embodiments, the erythrocyte precursor cells modified in accordance with the methods described herein are CD133+. In certain embodiments, the erythrocyte precursor cells modified in accordance with the methods described herein are CD34+/CD133+.

In certain embodiments, the erythrocyte precursor cells modified in accordance with the methods described herein are isolated from cells that have been isolated from a donor (e.g., a living or cadaveric donor). In certain embodiments, the erythrocyte precursor cells modified in accordance with the methods described herein are cells that have been isolated from a subject and cultured in vitro. Such cells can be passaged once or multiple times, e.g. twice, three times, four times, five times, six times, seven times, eight times, nine times, ten times, or more than ten times. In a specific embodiment, said primary cells have been passaged no more than six times.

In certain embodiments, the erythrocyte precursor cells modified in accordance with the methods described herein are differentiated in a manner that results in the production of an erythrocyte population that comprises a specific blood type, e.g., the erythrocyte precursor cells are differentiated in a manner that yields erythrocytes that are blood type O and Rh-. Methods for differentiating erythrocyte precursor cells in such fashion are known in the art (see, e.g., Miharada et al., Nat Biotechnol, 2006, 24(10):1255-6, the disclosure of which is hereby incorporated by reference in its entirety).

In certain embodiments, the erythrocyte precursor cells are modified and then expanded in culture. In other embodiments, the erythrocyte precursor cells are expanded in culture, and then modified.

### 4.2.2 Nucleic Acids

In accordance with the methods described herein, erythrocyte precursor cells are genetically engineered to express one or more nucleic acids (e.g., RNA, DNA, or synthetic polynucleotide) that are not normally expressed by said erythrocyte precursor cells, and thus are capable of producing a polypeptide(s) that is not normally present on or in erythrocytes.

In certain embodiments, the erythrocyte precursor cells are modified so that a polypeptide of interest, not normally present on or in the erythrocytes produced by said erythrocyte precursor cells, is present on or in said erythrocytes as a fusion protein that comprises said polypeptide of interest and a polypeptide that is normally present on or in said erythrocytes. Such fusion proteins can comprise, e.g., the whole of a polypeptide that is normally present on or in said erythrocytes, or a domain thereof (e.g., a transmembrane domain, intracellular domain, extracellular domain, or part thereof, or any combination thereof). Exemplary proteins that are expressed on or in erythrocytes that can be included in such fusion proteins include, without limitation, hemoglobin, transferrin receptor protein 1 (CD71), band3, aquaporin 1, Glut1, kidd antigen protein, rhAG, ATPases, Gardos Channel, ICAM-4, BCAM, Protein 4.1R, glycophorin A, glycophorin C, glycophorin D, CD55, CD59, XK, Duffy, Aducin, and Dematin. Other genes expressed by erythrocytes that could be used in the generation of fusion proteins are described in Kabanova et al., 2009, Int. J. Med. Sci. 6(4):159-159, the disclosure of which is incorporated by reference herein in its entirety.

In a specific embodiment, the erythrocyte precursor cells modified in accordance with the methods described herein are genetically engineered to express a nucleic acid that encodes an antigen. Expression of an antigen on or in a modified erythrocyte produced by said erythrocyte precursor cells can be utilized to generate an immune response against the antigen in a subject to which said modified erythrocytes have been administered.

In one embodiment, the erythrocyte precursor cells modified in accordance with the methods described herein are genetically engineered to express a nucleic acid that encodes a tumor antigen, i.e., an antigen that is expressed by one or more types of tumors/cancers. Tumor antigens include those antigens that are known to be expressed (or overexpressed) by cancerous cells. In a specific embodiment, tumor antigens are expressed on cancerous cells, but are not expressed by non-cancerous (e.g., normal) cells, i.e., the antigens are tumor-specific antigens. In another specific embodiment, tumor antigens are expressed on both cancerous and non-cancerous cells, i.e., the antigens are tumor-associated antigens. The erythrocyte precursor cells modified in accordance with the methods described herein can be genetically engineered to express a nucleic acid that encodes a tumor antigen associated with or specific to any type of cancer known to those of skill in the art including, without limitation, leukemia, lymphoma, myeloma, bone and connective tissue sarcomas, brain cancer, breast cancer, ovarian cancer, kidney cancer, pancreatic cancer, testicular cancer, esophageal cancer, stomach cancer, lung cancer (e.g., small cell lung cancer (SCLC), non-small cell lung cancer (NSCLC), and mesothelioma), liver cancer, throat cancer, skin cancer, glioma, glioblastoma, and prostate cancer. Exemplary tumor antigens include, without limitation, SART-1, SART-3, tenascin, 4Ff2, EGFR variant III (EGFRvIII), CA-125, alphafetoprotein (AFP), carcinoembryonic antigen (CEA), MUC-1, epithelial tumor antigen (ETA), tyrosinase, melanoma-associated antigen (MAGE), prostate-specific antigen (PSA), prostate stem cell antigen (PSCA), and cancer testis antigens (e.g., NY-ESO-1). Exemplary tumor antigens also include the abnormal products resulting from mutation of normally expressed proteins including, but not limited to, p53, ras, BRCA1, and BRCA2. In a specific embodiment, provided herein are modified erythrocytes produced by (e.g., via differentiation) the modified erythrocyte precursor cells genetically engineered to express a nucleic acid that encodes a tumor antigen, i.e., an antigen that is expressed by one or more types of tumors/cancers.

In another embodiment the erythrocyte precursor cells modified in accordance with the methods described herein are genetically engineered to express a nucleic acid that encodes a self antigen, wherein expression of the self antigen by erythrocytes produced by said erythrocyte precursor cells is sufficient to induce self-tolerance to said antigen in a subject to whom said erythrocytes are administered. The erythrocyte precursor cells modified in accordance with the methods described herein can be genetically engineered to express a nucleic acid that encodes an antigen associated with or specific to any type of allergy or autoimmune disease including, without limitation, Addison's disease, Behcet's disease, chronic active hepatitis, chronic fatigue immune dysfunction syndrome (CFIDS), chronic inflammatory demyelinating polyneuropathy, Churg-Strauss syndrome, inflammatory bowel disease (e.g., Crohn's disease), Graves' disease, Guillain-Barre, Myasthenia Gravis, Reiter's syndrome, rheumatoid arthritis, sarcoidosis, Sjögren's syndrome, systemic lupus erythematosus, hay fever, conjunctivitis, dermatitis, food allergies, seasonal allergies, drug allergies, and animal (e.g., pet) allergies. Exemplary antigens for which induction of self-tolerance in a subject may be desirable include, without limitation, peptides derived from insulin, glutamic acid decarboxylase 65 (GAD 65), heat shock protein 60 (HSP 60), hCDR1, HLA DR2, HLA DR3, HLA DR4, and PTPN22. Other exemplary antigens for which induction of self-tolerance in a subject may be desirable include, without limitation, peptide sequences derived encephalitogenic peptides (e.g., myelin basic protein) and peptide sequences derived from OmpC or other tologenic peptides associated with Crohn's disease. In a specific embodiment, provided herein are modified erythrocytes produced by (e.g., via differentiation) the modified erythrocyte precursor cells genetically engineered to express a nucleic acid that encodes a self antigen.

In another embodiment the erythrocyte precursor cells modified in accordance with the methods described herein are genetically engineered to express a nucleic acid that encodes a viral antigen, i.e., an antigen is expressed by one or more types of virus. The erythrocyte precursor cells modified in accordance with the methods described herein can be genetically engineered to express a nucleic acid that encodes an antigen from any known type of virus including, without limitation, respiratory syncytial virus (RSV), influenza virus (influenza A virus, influenza B virus, or influenza C virus), human metapneumovirus (HMPV), rhinovirus, parainfluenza virus, SARS Coronavirus, human immunodeficiency virus (HIV), hepatitis virus (A, B, C), ebola virus, herpes virus, rubella, variola major, and variola minor. In a specific embodiment, provided herein are modified erythrocytes produced by (e.g., via differentiation) the modified erythrocyte precursor cells genetically engineered to express a nucleic acid that encodes a viral antigen.

In another embodiment the erythrocyte precursor cells modified in accordance with the methods described herein are genetically engineered to express a nucleic acid that encodes a bacterial antigen, i.e., the antigen is expressed by one or more types of bacteria. The erythrocyte precursor cells modified in accordance with the methods described herein can be genetically engineered to express a nucleic acid that encodes an antigen from any known type of bacteria including, without limitation, *Streptococcus pneumoniae, Mycobacterium tuberculosis, Chlamydia pneumoniae, Bordetella pertussis, Mycoplasma pneumoniae, Haemophilus influenzae, Plasmodium falciparum, Moraxella catarrhalis, Legionella, Pneumocystis jiroveci , Chlamydia psittaci, Chlamydia trachomatis, Bacillus anthracis, Francisella tularensis, Staphylococcus aureus* (including, e.g., methicillin-resistant *Staphylococcus aureus), Borrelia burgdorferi, Borrelia afzelii, Borrelia garinii, rickettsia rickettsii, Salmonella, Yersinia pestis, Shigella, Escherichia coli, Corynebacterium diphtheriae,* and *Treponema pallidum.* In a specific embodiment, provided herein are modified erythrocytes produced by (e.g., via differentiation) the modified erythrocyte precursor cells genetically engineered to express a nucleic acid that encodes a bacterial antigen.

In certain embodiments, erythrocyte precursor cells that have been modified to express a nucleic acid that encodes an antigen are additionally engineered to express a nucleic acid that encodes an immunomodulatory protein, e.g., a protein that is capable of causing increased recognition/processing of the antigen expressed by the erythrocyte precursor cells. In a specific embodiment, the immunomodulatory protein is capable of activating at least one type of antigen-presenting cell (e.g., a professional antigen-presenting cell such as a dendritic cell or macrophage). In another specific embodiment, the immunomodulatory protein comprises a toll-like receptor modulator or a type-4 toll-like receptor agonist. Exemplary immunomodulatory proteins include, without limitation, galectin-1, galectin-3, LAG-3, interleukin-10, Tim-3, CD200, HVEM, B7.1/B7.2, B7H1, and B7DC. In a specific embodiment, provided herein are modified erythrocytes produced by (e.g., via differentiation) the modified erythrocyte precursor cells genetically engineered to express a nucleic acid that encodes an immunomodulatory protein.

In certain embodiments, erythrocyte precursor cells, e.g., erythrocyte precursor cells that have been modified to express a nucleic acid that encodes an antigen, are additionally engineered to express a nucleic acid that encodes a polypeptide that targets the erythrocyte produced by said erythrocyte precursor cells for phagocytosis including, but not limited to, calreticulin, phosphatidylserine, and danger- and pathogen-associated molecular pattern receptors (DAMP/PAMP). In a specific embodiment, provided herein are modified erythrocytes produced by (e.g., via differentiation) the modified erythrocyte precursor cells genetically engineered to express a nucleic acid that encodes an antigen and a polypeptide that targets the erythrocyte produced by said erythrocyte precursor cells for phagocytosis.

In certain embodiments, the modified erythrocyte precursor cells, and/or modified erythrocytes produced by such cells comprise an opsonin, i.e., the modified erythrocyte precursor cells and/or modified erythrocytes comprise (e.g., are engineered to express or have bound to them) a molecule that results in immune targeting of the modified erythrocyte precursor cells and/or modified erythrocytes. In specific embodiments, the opsonin targets the modified erythrocyte precursor cells and/or modified erythrocytes for phagocytosis, e.g., phagocytosis by macrophages. For example, a modified erythrocyte precursor cell provided herein can express a nucleic acid (e.g., an RNA, DNA, or a synthetic polynucleotide), e.g., an antigen, and further comprise an opsonin. In a specific embodiment, provided herein are modified erythrocytes that comprise a nucleic acid (e.g., an RNA, DNA, or a synthetic polynucleotide) and an opsonin. Exemplary opsonins include, without limitation, immunoglobulins (e.g., IgG, antibodies), molecules that activate the complement system (e.g., C3b, iC3b, and C1q), molecules that inactivate complement system inhibitors such as DAF and CD59 (e.g., molecules such as streptavidin that can induce clustering of complement system inhibitors), and molecules that activate natural killer cells (e.g., IL-12, IL-15, IL-18, IL-2, and CCL5).

In certain embodiments, the erythrocyte precursor cells modified in accordance with the methods described herein are genetically engineered to express a nucleic acid that encodes a polypeptide that performs at least one physiological function that is lacking in a subject to whom the erythrocytes produced by said modified erythrocyte precursor cells are administered. For example, the modified erythrocyte precursor cells may express a nucleic acid that encodes a cytokine, a hormone, proinsulin/insulin, or an insulin receptor. In a specific embodiment, provided herein are modified erythrocytes produced by (e.g., via differentiation) the modified erythrocyte precursor cells genetically engineered to express a nucleic acid that encodes a polypeptide that performs at least one physiological function that is lacking in a subject to whom the erythrocytes produced by said modified erythrocyte precursor cells are administered.

In a specific embodiment, the erythrocyte precursor cells modified in accordance with the methods described herein are genetically engineered to express a nucleic acid that encodes a cytokine or an active part thereof, wherein said cytokine or an active part thereof is present on or in erythrocytes produced by said erythrocyte precursor cells. Exemplary cytokines that may be produced by such modified erythrocyte precursor cells include, without limitation, adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), interferon alpha, interferon beta, interferon gamma, granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (P1GF), platelet-derived growth factor (PDGF), thrombopoietin (Tpo), transforming growth factor alpha (TGF-a), TGF-β, tumor necrosis factor alpha (TNF-a), or vascular endothelial growth factor (VEGF). In a specific embodiment, provided herein are modified erythrocytes produced by (e.g., via differentiation) the modified erythrocyte precursor cells genetically engineered to express a nucleic acid that encodes a cytokine or an active part thereof.

In another specific embodiment, the erythrocyte precursor cells modified in accordance with the methods described herein are genetically engineered to express a nucleic acid that encodes insulin or proinsulin, wherein said insulin or proinsulin is present on or in erythrocytes produced by said erythrocyte precursor cells. In another specific embodiment, the erythrocyte precursor cells modified in accordance with the methods described herein are genetically engineered to express a nucleic acid that encodes a receptor for insulin, wherein said insulin or proinsulin is present on or in erythrocytes produced by said erythrocyte precursor cells. In certain embodiments, said erythrocyte precursor cells also are genetically engineered to produce one or more of prohormone convertase 1, prohormone convertase 2, or carboxypeptidase E. In a specific embodiment, provided herein are modified erythrocytes produced by (e.g., via differentiation) the modified erythrocyte precursor cells genetically engineered to express a nucleic acid that encodes insulin or proinsulin.

In another specific embodiment, the erythrocyte precursor cells modified in accordance with the methods described herein are genetically engineered to express a nucleic acid that encodes a hormone or prohormone. Exemplary hormones that may be produced by such modified erythrocyte precursor cells include, without limitation, antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), and thyrotropin-releasing hormone (TRH). In a specific embodiment, provided herein are modified erythrocytes produced by (e.g., via differentiation) the modified erythrocyte precursor cells genetically engineered to express a nucleic acid that encodes a hormone.

In another specific embodiment, the erythrocyte precursor cells modified in accordance with the methods described herein are genetically engineered to express a nucleic acid that encodes an enzyme. Exemplary enzymes that may be produced by such modified erythrocyte precursor cells include, without limitation, asparaginase, alcohol dehydrogenase, glutamate dehydrogenase, rhodanase, or urate oxidase. In a specific embodiment, provided herein are modified erythrocytes produced by (e.g., via differentiation) the modified erythrocyte precursor cells genetically engineered to express a nucleic acid that encodes an enzyme.

In another specific embodiment, the erythrocyte precursor cells modified in accordance with the methods described herein are genetically engineered to express a nucleic acid that encodes a first protein that is capable of binding to a second protein, wherein said first protein is expressed at the surface of erythrocytes produced by said modified erythrocyte precursor cells, and wherein said protein is linked (e.g., via a cleavable linker) to a desired third protein. In certain embodiments, the first protein is an antibody (e.g., a single chain antibody) and the second protein is an antigen. In certain embodiments, the first protein is an antigen and the second protein is an antibody. In certain embodiments, the first protein is a ligand and the second protein is a receptor for said ligand. In certain embodiments, the first protein is a receptor and the second protein is a ligand for said receptor. In specific embodiments, the erythrocytes produced by such modified erythrocyte precursor cells can be administered to a subject and serve as a delivery mechanism for the third protein. For example, prior to administration of modified erythrocytes comprising the first protein to a subject, the second/third protein fusion can be cultured with the modified erythrocytes, so as to induce binding between the first and second proteins. Thus, upon administration to said subject, the modified erythrocytes comprise the first protein linked to the second/third protein fusion. In certain embodiments, the second protein and the third protein are linked by a cleavable linker, so that the third protein can be released at a desired location in the subject to which the erythrocytes produced by said modified erythrocyte precursor cells are administered. For example, in embodiments where it is desirable for the third protein to be released in the bloodstream of a subject, a cleavable linker that is cleaved by a component (e.g., a protease) naturally found in the subject's bloodstream can be selected.

In certain embodiments, the modified erythrocyte precursor cells are engineered to express a polypeptide that is a first member of a binding pair, wherein said binding pair comprises a first member and a second member, e.g., such that erythrocytes differentiated from such erythrocyte precursors comprise said first member of a binding pair on their surface. In such embodiments, a polypeptide comprising the second member of said binding pair can be contacted with the modified erythrocytes, such that said polypeptide comprising said second member of said binding pair is bound to at least a portion, or substantially all, of the first member of said binding pair on said erythrocytes. In certain embodiments, said second member of said binding pair is a ligand, and said first member of said binding pair is a receptor or ligand-binding domain of a receptor. In other embodiments, said first member of said binding pair is a ligand, and said second member of said binding pair is a receptor or ligand-binding domain of a receptor. In certain embodiments, the second member of the binding pair is part of a fusion protein that comprises at least one other polypeptide or protein domain. In some embodiments, therefore, the modified erythrocyte can be a part of a therapeutic combination comprising the modified erythrocyte comprising the first member of the binding pair, and a second protein that is a fusion protein that comprises the second member of said binding pair and a polypeptide of interest. When the fusion protein is contacted with the modified erythrocyte, the modified erythrocyte becomes partially or substantially completely coated with the polypeptide of interest, becoming a delivery vehicle for the polypeptide of interest. Without being bound by any particular mechanism or theory, such modified erythrocytes can be utilized to administer a polypeptide of interest, wherein the polypeptide of interest has improved in vivo pharmacokinetic properties and/or does not present, or has minimized, systemic toxicity that is associated with the polypeptide when it is not administered as part of a binding pair.

In certain embodiments, the modified erythrocyte precursor cells described herein may be further engineered to "knock out" or "knock down" expression of one or more genes of the erythrocyte precursor cell itself or of another cell. The expression of a gene native to an erythrocyte precursor cell can be diminished by, for example, inhibition of expression by inactivating the gene completely by, e.g., homologous recombination. In one embodiment, for example, an exon encoding an important region of the protein, or an exon 5' to that region, is interrupted by a positive selectable marker, e.g., neo, preventing the production of normal mRNA from the target gene and resulting in inactivation of the gene. A gene may also be inactivated by creating a deletion in part of a gene or by deleting the entire gene. By using a construct with two regions of homology to the target gene that are far apart in the genome, the sequences intervening the two regions can be deleted (Mombaerts et al., 1991, Proc. Nat. Acad. Sci. U.S.A. 88:3084). Antisense, DNAzymes, small interfering RNA, and ribozyme molecules that inhibit expression of a target gene can also be used to reduce the level of target gene activity. For example, antisense RNA molecules which inhibit the expression of major histocompatibility gene complexes (HLA) have been shown to be versatile with respect to immune responses of a subject to administration of a cell population (e.g., transfusion of erythrocytes derived from erythrocyte precursor cells). In accordance with such embodiments, the expression of genes native to an erythrocyte precursor cell that encode proteins having antigenic properties (e.g., Rh proteins) can be knocked down or knocked out, thus resulting in the production of erythrocytes from said erythrocyte precursor cells that have reduced antigenicity. In a specific embodiment, the gene that encodes hemoglobin is knocked down or knocked out in the modified erythrocyte precursor cells, such that modified erythrocytes produced by said modified erythrocyte precursor cells do not comprise hemoglobin or have a reduced hemoglobin content as compared to normal erythrocytes.

In certain embodiments, the modified erythrocyte precursor cells described herein may be further engineered to express a nucleic acid molecule comprising a nucleotide sequence that encodes a protein that allows the erythrocyte precursor cells to proliferate for greater than 5, 10, 20, 30, 40, 50 or more population doublings. In a specific embodiment, the erythrocyte precursor cells are engineered to express a growth-promoting polypeptide, e.g., v-myc, N-myc, c-myc, p53, SV40 large T antigen, polyoma large T antigen, E1a adenovirus or E7 protein of human papillomavirus..

In certain embodiments, the modified erythrocyte precursor cells described herein may be further engineered to express a nucleic acid molecule comprising a nucleotide sequence encoding a polypeptide/protein of interest, wherein expression of the polypeptide/protein of interest is controllable by an exogenous factor, e.g., polypeptide, small organic molecule, or the like. Therefore, in another embodiment, the nucleic acid used to genetically engineer an erythrocyte precursor cell can comprise nucleotide sequences encoding a first polypeptide and a second polypeptide, wherein said first and second polypeptides are expressed as a fusion protein that is translationally upregulated in the presence of an exogenous factor. The polypeptide can be expressed transiently or long-term (e.g., over the course of weeks or months). Such a nucleic acid molecule can additionally comprise a nucleotide sequence encoding a polypeptide that allows for positive selection of engineered erythrocyte precursor cells, or allows for visualization of the engineered erythrocyte precursor cells. In another embodiment, the nucleotide sequence encodes a polypeptide that is, *e.g.,* fluorescent under appropriate visualization conditions, *e.g.,* luciferase (Luc).

### 4.2.3 Erythrocytes Produced by Modified Erythrocyte Precursor Cells

Also provided herein are erythrocytes produced by the modified erythrocyte precursor cells described herein, i.e., modified erythrocytes. The modified erythrocytes produced from the modified erythrocyte precursor cells described herein are produced in a manner that results in the polypeptide expressed by the modified erythrocyte precursor cells that is not normally present on or in erythrocytes to be present on or in the modified erythrocytes. In certain embodiments, the polypeptide expressed by the modified erythrocyte precursor cells that is not normally present in or in erythrocytes is one that is present in or on the modified erythrocytes at a level 10-, 20-, 30-, 40-, 50-, 100-, or 500-fold higher than in or on wild-type erythrocytes. In certain embodiments, the polypeptide is a fusion protein between an erythrocyte-specific protein (i.e., a protein known to normally be present on or in erythrocytes) and a polypeptide that is not normally present on or in erythrocytes.

In certain embodiments, the modified erythrocytes described herein are blood type O. In certain embodiments, the modified erythrocytes described herein are blood type A. In certain embodiments, the modified erythrocytes described herein are blood type B. In certain embodiments, the modified erythrocytes described herein are blood type AB. In certain embodiments, the modified erythrocytes described herein are blood type Rh+. In certain embodiments, the modified erythrocytes described herein are blood type Rh-. In certain embodiments, the modified erythrocytes described herein are blood type O and also are Rh-.

In certain embodiments, the polypeptide that is not normally present on or in erythrocytes, but is present on or in the modified erythrocytes described herein, is a membrane-bound protein. In certain embodiments, the polypeptide that is not normally present on or in erythrocytes, but is present on or in the modified erythrocytes described herein, is a soluble protein contained within the interior of said erythrocytes.

In certain embodiments, the polypeptide that is not normally present on or in erythrocytes, but is present on or in the modified erythrocytes described herein, is a polypeptide that performs at least one physiological function that is lacking in a subject to whom the modified erythrocyte is administered. In another specific embodiment, the polypeptide is a hormone. In another specific embodiment, the polypeptide is insulin.

In certain embodiments, the polypeptide that is not normally present on or in erythrocytes, but is present on or in the modified erythrocytes described herein, is a polypeptide that possesses one or more activities that confer a therapeutic benefit in a subject to whom the modified erythrocyte is administered. In a specific embodiment, the polypeptide is a cytokine.

In certain embodiments, the polypeptide that is not normally present on or in erythrocytes, but is present on or in the modified erythrocytes described herein, is a tumor antigen. In certain embodiments, the polypeptide that is not normally present on or in erythrocytes, but is present on or in the modified erythrocytes described herein, is a viral antigen. In certain embodiments, the polypeptide that is not normally present on or in erythrocytes, but is present on or in the modified erythrocytes described herein, is a bacterial antigen. In certain embodiments, the polypeptide that is not normally present on or in erythrocytes, but is present on or in the modified erythrocytes described herein, is an antigen capable of inducing self-tolerance to the antigen in a subject to whom the modified erythrocytes are administered.

In certain embodiments, the modified erythrocytes described herein comprise an immunomodulatory polypeptide. In certain embodiments, the modified erythrocytes described herein comprise a polypeptide that confers one or more properties to said modified erythrocyte precursor cells that is not normally associated with the cells, e.g., the polypeptide makes the modified erythrocyte precursor cells more susceptible to phagocytosis or possess a longer life span (e.g., can undergo more population doublings) than unmodified erythrocyte precursor cells.

Erythrocytes may be differentiated from erythrocyte precursor cells by any art-accepted methods, including, e.g., the methods described in U.S. patent application publication NO. 2011/0003387, the contents of which are hereby incorporated by reference in their entirety.

### 4.3 COMPOSITIONS

Provided herein are compositions comprising the modified erythrocyte precursor cells described herein. Also provided herein are compositions comprising erythrocytes produced by the modified erythrocyte precursor cells described herein. In certain embodiments, such compositions are formulated for use in vivo.

In certain embodiments, the compositions provided herein comprise modified erythrocyte precursor cells described herein and/or modified erythrocytes produced from such cells and a pharmaceutically-acceptable carrier, *e.g.,* a saline solution or other accepted physiologically-acceptable solution for in vivo administration, e.g., transfusion.

In certain embodiments, the pharmaceutical compositions provided herein comprise any number of modified erythrocyte precursor cells described herein or modified erythrocytes produced from such cells. For example, a single unit dose of modified erythrocyte precursor cells described herein or modified erythrocytes produced from such cells can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more modified erythrocyte precursor cells described herein or modified erythrocytes produced from such cells.

In certain embodiments, the pharmaceutical compositions provided herein comprise modified erythrocytes and unmodified erythrocytes. For example, a single unit dose of erythrocytes (e.g., modified and unmodified erythrocytes) can comprise, in various embodiments, about, at least, or no more than at least 10%, 20%, 30%, 40%, 50%, 60%, 70%, 75%, 80%, 85%, 90%, 95%, or 99% modified erythrocytes, wherein the remaining erythrocytes in the composition are unmodified erythrocytes.

In certain embodiments, the pharmaceutical compositions provided herein comprise populations of modified erythrocyte precursor cells described herein or modified erythrocytes produced from such cells that comprise at least 50% viable modified erythrocyte precursor cells described herein or modified erythrocytes produced from such cells, or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

In a specific embodiment, provided herein are pharmaceutical compositions comprising modified erythrocytes. In one embodiment, at least 10%, 20%, 30%, 40%, 50%, 60%, 70%, 75%, 80%, 85%, 90%, 95%, or 99% of the modified erythrocytes in said compositions are erythrocytes produced from the modified erythrocyte precursor cells described herein. In another embodiment, at least 10% to 20%, 20% to 30%, 30% to 40%, 40% to 50%, 50% to 60%, 60% to 70% to 80%, 80% to 90%, or 90% to 99% of the modified erythrocytes in said compositions are erythrocytes produced from the modified erythrocyte precursor cells described herein.

### 4.4 USES

In certain embodiments, the modified erythrocyte precursor cells described herein or modified erythrocytes produced from such cells are used to treat a subject having a disease or disorder caused by a genetic defect resulting in the loss of at least one protein function, wherein said protein function is at least partly complemented by said erythrocytes, and wherein the defect is not an erythrocyte defect.

In a specific embodiment, the modified erythrocyte precursor cells express a hormone, and said hormone is present on or in modified erythrocytes produced by said erythrocyte precursor cells. In a specific embodiment, provided herein is a method of a hormone deficiency in a subject in need thereof, comprising administering to said subject erythrocytes produced by modified erythrocyte precursor cells, wherein said modified erythrocyte precursor cells express a nucleic acid that encodes a hormone, and wherein said hormone is present on or in said modified erythrocytes.

In another specific embodiment, the modified erythrocyte precursor cells express insulin, and insulin is present on or in modified erythrocytes produced by said erythrocyte precursor cells. In a specific embodiment, provided herein is a method of treating diabetes in a subject in need thereof, comprising administering to said subject erythrocytes produced by modified erythrocyte precursor cells, wherein said modified erythrocyte precursor cells express a nucleic acid that encodes insulin, and wherein said insulin is present on or in said modified erythrocytes.

In certain embodiments, the modified erythrocyte precursor cells described herein or modified erythrocytes produced from such cells are used to treat a subject having a disease or disorder that can be treated by the administration of one or more cytokines. In a specific embodiment, provided herein is a method of treating neutropenia in a subject in need thereof, comprising administering to said subject modified erythrocytes produced by modified erythrocyte precursor cells, wherein said modified erythrocyte precursor cells express a nucleic acid that encodes granulocyte colony stimulating factor (G-CSF), and wherein said G-CSF is present on or in said modified erythrocytes. In another specific embodiment, provided herein is a method of treating a viral infection in a subject in need thereof, comprising administering to said subject modified erythrocytes produced by modified erythrocyte precursor cells, wherein said modified erythrocyte precursor cells express a nucleic acid that encodes interferon alpha, and wherein said interferon alpha is present on or in said modified erythrocytes. In another specific embodiment, provided herein is a method of treating cancer in a subject in need thereof, comprising administering to said subject modified erythrocytes produced by modified erythrocyte precursor cells, wherein said modified erythrocyte precursor cells express a nucleic acid that encodes a cytokine known to be efficacious in the treatment of cancer, and wherein said cytokine is present on or in said modified erythrocytes.

In certain embodiments, the modified erythrocyte precursor cells described herein or modified erythrocytes produced from such cells are used to treat a subject having cancer by way of introducing a tumor antigen into said subject using modified erythrocytes. In a specific embodiment, provided herein is a method of treating cancer in a subject in need thereof, comprising administering to said subject modified erythrocytes produced by modified erythrocyte precursor cells, wherein said modified erythrocyte precursor cells express a nucleic acid that encodes a tumor antigen, and wherein said tumor antigen is present on or in said modified erythrocytes. In a specific embodiment, said tumor antigen is an antigen specific to or associated with leukemia, lymphoma, myeloma, bone and connective tissue sarcomas, brain cancer, breast cancer, ovarian cancer, kidney cancer, pancreatic cancer, testicular cancer, esophageal cancer, stomach cancer, lung cancer (e.g., small cell lung cancer (SCLC), non-small cell lung cancer (NSCLC), and mesothelioma), liver cancer, throat cancer, skin cancer, or prostate cancer; wherein said administration of erythrocytes comprising said antigen results in treatment of the cancer. In another specific embodiment, said cancer antigen is CA-125, alphafetoprotein (AFP), carcinoembryonic antigen (CEA), MUC-1, epithelial tumor antigen (ETA), tyrosinase, melanoma-associated antigen (MAGE), the abnormal product resulting from mutation of ras, the abnormal product resulting from mutation of p53, prostate-specific antigen (PSA), prostate stem cell antigen (PSCA), the abnormal product resulting from mutation BRCA1, and the abnormal product resulting from mutation BRCA2.

In certain embodiments, the modified erythrocyte precursor cells described herein or modified erythrocytes produced from such cells are used to treat a subject having an allergy or autoimmune disease, by way of introducing an antigen capable of inducing self-tolerance in a subject to whom said antigen is administered using modified erythrocytes. In a specific embodiment, provided herein is a method of treating an allergy or autoimmune disease in a subject in need thereof, comprising administering to said subject modified erythrocytes produced by modified erythrocyte precursor cells, wherein said modified erythrocyte precursor cells express a nucleic acid that encodes an antigen capable of inducing self-tolerance to the antigen in a subject to whom the modified erythrocytes are administered, and wherein said antigen is present on or in said modified erythrocytes. In a specific embodiment, said antigen capable of inducing self-tolerance is an antigen specific to or associated with Addison's disease, Behcet's disease, chronic active hepatitis, chronic fatigue immune dysfunction syndrome (CFIDS), chronic inflammatory demyelinating polyneuropathy, Churg-Strauss syndrome, inflammatory bowel disease (e.g., Crohn's disease), Graves' disease, Guillain-Barre, Myasthenia Gravis, Reiter's syndrome, rheumatoid arthritis, sarcoidosis, Sjögren's syndrome, systemic lupus erythematosus, hay fever, conjunctivitis, dermatitis, food allergies, seasonal allergies, drug allergies, and animal (e.g., pet) allergies; wherein said administration of erythrocytes comprising said antigen results in treatment of the allergy or autoimmune disease. In another specific embodiment, said cancer antigen is a peptide derived from insulin, glutamic acid decarboxylase 65 (GAD 65), heat shock protein 60 (HSP 60), hCDR1, HLA DR2, HLA DR3, HLA DR4, or PTPN22.

In certain embodiments, the modified erythrocyte precursor cells described herein or modified erythrocytes produced from such cells are used to treat a subject having a viral disease or infection, by way of introducing a viral antigen into said subject using modified erythrocytes. In a specific embodiment, provided herein is a method of treating a viral disease or infection in a subject in need thereof, comprising administering to said subject modified erythrocytes produced by modified erythrocyte precursor cells, wherein said modified erythrocyte precursor cells express a nucleic acid that encodes a viral antigen, and wherein said viral antigen is present on or in said modified erythrocytes. In a specific embodiment, said viral antigen is an antigen derived from a protein of respiratory syncytial virus (RSV), influenza virus (influenza A virus, influenza B virus, or influenza C virus), human metapneumovirus (HMPV), rhinovirus, parainfluenza virus, SARS Coronavirus, human immunodeficiency virus (HIV), hepatitis virus (A, B, C), ebola virus, herpes virus, rubella, variola major, or variola minor; wherein said administration of erythrocytes comprising said antigen results in treatment of the viral disease or infection.

In certain embodiments, the modified erythrocyte precursor cells described herein or modified erythrocytes produced from such cells are used to treat a subject having a bacterial disease or infection, by way of introducing a bacterial antigen into said subject using modified erythrocytes. In a specific embodiment, provided herein is a method of treating a bacterial disease or infection in a subject in need thereof, comprising administering to said subject modified erythrocytes produced by modified erythrocyte precursor cells, wherein said modified erythrocyte precursor cells express a nucleic acid that encodes a bacterial antigen, and wherein said bacterial antigen is present on or in said modified erythrocytes. In a specific embodiment, said bacterial antigen is an antigen derived from a protein of *Streptococcus pneumoniae, Mycobacterium tuberculosis, Chlamydia pneumoniae, Bordetella pertussis, Mycoplasma pneumoniae, Haemophilus influenzae, Plasmodium falciparum, Moraxella catarrhalis, Legionella, Pneumocystis jiroveci , Chlamydia psittaci, Chlamydia trachomatis, Bacillus anthracis, Francisella tularensis, Borrelia burgdorferi, Borrelia afzelii, Borrelia garinii, rickettsia rickettsii, Salmonella, Yersinia pestis, Shigella, Escherichia coli, Corynebacterium diphtheriae,* and *Treponema pallidum;* wherein said administration of erythrocytes comprising said antigen results in treatment of the bacterial disease or infection.

In certain embodiments, the modified erythrocyte precursor cells described herein or modified erythrocytes produced from such cells are used to treat a subject having an enzyme deficiency (e.g., the subject does not make enough or makes a deficient/defective version of an enzyme), by way of introducing an enzyme into said subject using modified erythrocytes. In a specific embodiment, provided herein is a method of treating an enzyme deficiency in a subject in need thereof, comprising administering to said subject modified erythrocytes produced by modified erythrocyte precursor cells, wherein said modified erythrocyte precursor cells express a nucleic acid that encodes an enzyme, and wherein said enzyme is present on or in said modified erythrocytes. In another specific embodiment, the erythrocyte precursor cells modified in accordance with the methods described herein are genetically engineered to express a nucleic acid that encodes an enzyme. In a specific embodiment, said enzyme is asparaginase, alcohol dehydrogenase, glutamate dehydrogenase, rhodanase, or urate oxidase.

In certain embodiments, a subject to which erythrocytes produced from the modified erythrocyte precursor cells described herein are administered is an animal. In certain embodiments, the animal is a bird. In certain embodiments, the animal is a canine. In certain embodiments, the animal is a feline. In certain embodiments, the animal is a horse. In certain embodiments, the animal is a cow. In certain embodiments, the animal is a mammal, e.g., a horse, swine, mouse, or primate, preferably a human. In a specific embodiment, a subject to which erythrocytes produced from the modified erythrocyte precursor cells described herein are administered is a human.

### 4.5 KITS

Provided herein is a pharmaceutical pack or kit comprising one or more containers filled with one or more of the ingredients of the compositions described herein. Optionally associated with such container(s) can be a notice in the form prescribed by a governmental agency regulating the manufacture, use or sale of pharmaceuticals or biological products, which notice reflects approval by the agency of manufacture, use or sale for human administration.

In a specific embodiment, a kit provided herein comprises a composition comprising erythrocytes produced from the modified erythrocyte precursor cells described herein. Such a kit may optionally comprise a composition comprising one or more additional components. The kits encompassed herein can be used in accordance with the methods described herein.

The compositions and methods disclosed herein are not to be limited in scope by the specific embodiments described herein. Indeed, various modifications of the compositions and methods in addition to those described will become apparent to those of skill in the art from the foregoing description. Such modifications are intended to fall within the scope of the appended claims.

Various publications, patents and patent applications are cited herein, the disclosures of which are incorporated by reference in their entireties.

The following numbered paragraphs (paras) contain statements of broad combinations of the inventive technical features herein disclosed:-
1. A mammalian erythrocyte precursor cell genetically engineered to express a nucleic acid that encodes a polypeptide that is not normally present on or in erythrocytes, wherein said polypeptide comprises an antigen.
2. A mammalian erythrocyte precursor cell genetically engineered to express a nucleic acid that encodes a polypeptide that is not normally present on or in erythrocytes, wherein said protein is a cytokine, a hormone, or insulin.
3. The mammalian erythrocyte precursor cell of para 1, wherein said antigen is a tumor antigen, a viral antigen, or a bacterial antigen.
4. The mammalian erythrocyte precursor cell of para 1, wherein said antigen is an antigen capable of inducing self-tolerance to the antigen in a subject to whom the antigen is administered.
5. The mammalian erythrocyte v cell of para 3, wherein said tumor antigen is an antigen specific to or associated with leukemia, lymphoma, myeloma, bone and connective tissue sarcomas, brain cancer, breast cancer, ovarian cancer, kidney cancer, pancreatic cancer, testicular cancer, esophageal cancer, stomach cancer, lung cancer, liver cancer, throat cancer, skin cancer, or prostate cancer.
6. The mammalian erythrocyte precursor cell of para 3, wherein said tumor antigen is CA-125, alphafetoprotein (AFP), carcinoembryonic antigen (CEA), MUC-1, epithelial tumor antigen (ETA), tyrosinase, melanoma-associated antigen (MAGE), a protein resulting from mutation of ras, a protein resulting from mutation of p53, prostate-specific antigen (PSA), prostate stem cell antigen (PSCA), a protein resulting from mutation of BRCA1, or a protein resulting from mutation of BRCA2.
7. The mammalian erythrocyte precursor cell of para 3, wherein said viral antigen is an antigen derived from a protein of respiratory syncytial virus (RSV), influenza virus (influenza A virus, influenza B virus, or influenza C virus), human metapneumovirus (HMPV), rhinovirus, parainfluenza virus, SARS Coronavirus, human immunodeficiency virus (HIV), hepatitis virus (A, B, C), ebola virus, herpes virus, rubella, variola major, or variola minor.
8. The mammalian erythrocyte precursor cell of para 3, wherein said bacterial antigen is an antigen derived from a protein of *Streptococcus pneumoniae, Mycobacterium tuberculosis, Chlamydia pneumoniae, Bordetella pertussis, Mycoplasma pneumoniae, Haemophilus influenzae, Plasmodium falciparum, Moraxella catarrhalis, Legionella, Pneumocystis jiroveci* , *Chlamydia psittaci, Chlamydia trachomatis, Bacillus anthracis, Francisella tularensis, Borrelia burgdorferi, Borrelia afzelii, Borrelia garinii, rickettsia rickettsii, Salmonella, Yersinia pestis, Shigella, Escherichia coli, Corynebacterium diphtheriae,* and *Treponema pallidum.*
9. The mammalian erythrocyte precursor cell of para 4, wherein said antigen capable of inducing tolerance in a subject to whom the antigen is administered is an antigen derived from a protein known to be associated with an allergy or an autoimmune disease.
10. The mammalian erythrocyte precursor cell of para 9, wherein said an allergy or autoimmune disease is Addison's disease, Behcet's disease, chronic active hepatitis, chronic fatigue immune dysfunction syndrome (CFIDS), chronic inflammatory demyelinating polyneuropathy, Churg-Strauss syndrome, inflammatory bowel disease (e.g., Crohn's disease), Graves' disease, Guillain-Barre, Myasthenia Gravis, Reiter's syndrome, rheumatoid arthritis, sarcoidosis, Sjögren's syndrome, systemic lupus erythematosus, hay fever, conjunctivitis, dermatitis, food allergies, seasonal allergies, drug allergies, or animal allergies.
11. The mammalian erythrocyte precursor cell of para 9, wherein said antigen is a peptide derived from insulin, glutamic acid decarboxylase 65 (GAD 65), heat shock protein 60 (HSP 60), hCDR1, HLA DR2, HLA DR3, HLA DR4, or PTPN22.
12. The mammalian erythrocyte precursor cell of para 2, wherein said cytokine is adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), interferon alpha, interferon beta, interferon gamma, granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (PlGF), platelet-derived growth factor (PDGF), thrombopoietin (Tpo), transforming growth factor alpha (TGF-a), TGF-β, tumor necrosis factor alpha (TNF-a), or vascular endothelial growth factor (VEGF).
13. The mammalian erythrocyte precursor cell of para 2, wherein said hormone is antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), or thyrotropin-releasing hormone (TRH).
14. The mammalian erythrocyte precursor cell of any one of paras 1 to 13, wherein said polypeptide is a fusion protein with at least one domain of an erythrocyte-specific protein.
15. The mammalian erythrocyte precursor cell of para 14, wherein said erythrocyte-specific protein is hemoglobin, transferrin receptor protein 1 (CD71), band3, aquaporin 1, Glut1, kidd antigen protein, rhAG, ATPases, Gardos Channel, ICAM-4, BCAM, Protein 4.1R, glycophorin C, glycophorin D, XK, Duffy, Aducin, and Dematin.
16. The mammalian erythrocyte precursor cell of any one of paras 1 to 15, wherein said cell is further engineered to express a nucleic acid that encodes an immunomodulatory polypeptide.
17. The mammalian erythrocyte precursor cell of para 16, wherein said immunomodulatory polypeptide activates antigen presenting cells.
18. The mammalian erythrocyte precursor cell of para 16, wherein said immunomodulatory polypeptide comprises a toll-like receptor modulator.
19. The mammalian erythrocyte precursor cell of para 16, wherein said immunomodulatory polypeptide comprises a type-4 toll-like receptor agonist.
20. The mammalian erythrocyte precursor cell of para 16, wherein said immunomodulatory polypeptide is galectin-1, galectin-3, LAG-3, or interleukin-10.
21. The mammalian erythrocyte precursor cell of any one of paras 1 to 20, wherein said cell is further engineered to express a nucleic acid that encodes a polypeptide that targets the cell for phagocytosis.
22. The mammalian erythrocyte precursor cell of any one of paras 1 to 21, wherein said cell is further engineered to express at least one growth-promoting protein.
23. The mammalian erythrocyte precursor cell of para 22, wherein said growth-promoting protein is v-myc, N-myc, c-myc, p53, SV40 large T antigen, polyoma large T antigen, E1a adenovirus or E7 protein of human papillomavirus.
24. Modified erythrocytes produced by the mammalian precursor cell of any one of paras 1 to 23.
25. The modified erythrocytes of para 24, wherein the erythrocytes are type O.
26. The modified erythrocytes of para 24, wherein the erythrocytes are type A, type B, or type AB.
27. The modified erythrocytes of any one of paras 24 to 26, wherein the erythrocytes are Rh negative.
28. The modified erythrocytes of any one of paras 24 to 26, wherein the erythrocytes are Rh positive.
29. The modified erythrocytes of para 24, wherein the erythrocytes are:
   (a) blood type M, blood type N, blood type S, or blood type s;
   (b) blood type P1;
   (c) blood type Lua, blood type Lub, or blood type Lu(a);
   (d) blood type K (Kell), k (cellano), Kpa, Kpb, K(a+), Kp(a-b-) or K-k-Kp(a-b-);
   (e) blood type Le(a-b-), Le(a+b-) or Le(a-b+);
   (f) blood type Fy a, Fy b or Fy(a-b-); or
   (g) blood type Jk(a-b-), Jk(a+b-), Jk(a-b+) or Jk(a+b+).
30. The modified erythrocytes of any one of paras 24 to 29, wherein said polypeptide that is not normally present on or in erythrocytes is a membrane-bound polypeptide.
31. The modified erythrocytes of any one of paras 24 to 29, wherein said polypeptide that is not normally present on or in erythrocytes is a soluble protein contained within the interior of said erythrocyte.
32. A method of treating cancer in a subject in need thereof, comprising administering to the subject a modified erythrocyte produced by the mammalian erythrocyte precursor cell of paras 5 to 6.
33. A method of treating a viral disease or infection in a subject in need thereof, comprising administering to the subject a modified erythrocyte produced by the mammalian erythrocyte precursor cell of para 7.
34. A method of treating a bacterial disease or infection in a subject in need thereof, comprising administering to the subject a modified erythrocyte produced by the mammalian erythrocyte precursor cell of para 8.
35. A method of inducing tolerance to a self antigen in a subject in need thereof, comprising administering to the subject a modified erythrocyte produced by the mammalian erythrocyte precursor cell of paras 10 or 11.

## Claims

1. A mammalian erythrocyte precursor cell genetically engineered to express a nucleic acid that encodes a polypeptide that is not normally present on or in erythrocytes, wherein said polypeptide comprises an antigen, a cytokine, a hormone, or insulin.

2. The mammalian erythrocyte precursor cell of claim 1, wherein said antigen is a tumor antigen, a viral antigen, or a bacterial antigen.

3. The mammalian erythrocyte precursor cell of claim 1, wherein said antigen is an antigen capable of inducing self-tolerance to the antigen in a subject to whom the antigen is administered.

4. The mammalian erythrocyte precursor cell of claim 2, wherein said tumor antigen is:
(a) an antigen specific to or associated with leukemia, lymphoma, myeloma, bone and connective tissue sarcomas, brain cancer, breast cancer, ovarian cancer, kidney cancer, pancreatic cancer, testicular cancer, esophageal cancer, stomach cancer, lung cancer, liver cancer, throat cancer, skin cancer, or prostate cancer; or
(b) CA-125, alphafetoprotein (AFP), carcinoembryonic antigen (CEA), MUC-1, epithelial tumor antigen (ETA), tyrosinase, melanoma-associated antigen (MAGE), a protein resulting from mutation of ras, a protein resulting from mutation of p53, prostate-specific antigen (PSA), prostate stem cell antigen (PSCA), a protein resulting from mutation of BRCA1, or a protein resulting from mutation of BRCA2.

5. The mammalian erythrocyte precursor cell of claim 2, wherein said viral antigen is an antigen derived from a protein of respiratory syncytial virus (RSV), influenza virus (influenza A virus, influenza B virus, or influenza C virus), human metapneumovirus (HMPV), rhinovirus, parainfluenza virus, SARS Coronavirus, human immunodeficiency virus (HIV), hepatitis virus (A, B, C), ebola virus, herpes virus, rubella, variola major, or variola minor.

6. The mammalian erythrocyte precursor cell of claim 2, wherein said bacterial antigen is an antigen derived from a protein from *Streptococcus pneumoniae, Mycobacterium tuberculosis, Chlamydia pneumoniae, Bordetella pertussis, Mycoplasma pneumoniae, Haemophilus influenzae, Plasmodium falciparum, Moraxella catarrhalis, Legionella, Pneumocystisjiroveci, Chlamydia psittaci, Chlamydia trachomatis, Bacillus anthracis, Francisella tularensis, Borrelia burgdorferi, Borrelia afzelii, Borrelia garinii, rickettsia rickettsii, Salmonella, Yersinia pestis, Shigella, Escherichia coli, Corynebacterium diphtheriae,* or *Treponema pallidum.*

7. The mammalian erythrocyte precursor cell of claim 3, wherein said antigen capable of inducing tolerance in a subject to whom the antigen is administered is an antigen derived from a protein known to be associated with an allergy or an autoimmune disease,
preferably wherein said allergy or autoimmune disease is Addison's disease, Behcet's disease, chronic active hepatitis, chronic fatigue immune dysfunction syndrome (CFIDS), chronic inflammatory demyelinating polyneuropathy, Churg-Strauss syndrome, inflammatory bowel disease (e.g., Crohn's disease), Graves' disease, Guillain-Barre, Myasthenia Gravis, Reiter's syndrome, rheumatoid arthritis, sarcoidosis, Sjogren's syndrome, systemic lupus erythematosus, hay fever, conjunctivitis, dermatitis, food allergies, seasonal allergies, drug allergies, or animal allergies; or
preferably wherein said antigen is a peptide derived from insulin, glutamic acid decarboxylase 65 (GAD 65), heat shock protein 60 (HSP 60), hCDR1, HLA DR2, HLA DR3, HLA DR4, or PTPN22.

8. The mammalian erythrocyte precursor cell of claim 1, wherein said cytokine is adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), interferon alpha, interferon beta, interferon gamma, granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (P1GF), platelet-derived growth factor (PDGF), thrombopoietin (Tpo), transforming growth factor alpha (TGF-a), TGF-13, tumor necrosis factor alpha (TNF-a), or vascular endothelial growth factor (VEGF);
or wherein said hormone is antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), or thyrotropin-releasing hormone (TRH).

9. The mammalian erythrocyte precursor cell of any one of claims 1 to 8, wherein said polypeptide is a fusion protein with at least one domain of an erythrocyte-specific protein, optionally wherein said erythrocyte-specific protein is preferably hemoglobin, transferrin receptor protein 1 (CD71), band3, aquaporin 1, Glut 1, kidd antigen protein, rhAG, ATPases, Gardos Channel, ICAM-4, BCAM, Protein 4.1R, glycophorin C, glycophorin D, XK, Duffy, Aducin, and Dematin.

10. The mammalian erythrocyte precursor cell of any one of claims 1 to 9, wherein said cell is further engineered:
(i) to express a nucleic acid that encodes an immunomodulatory polypeptide, preferably wherein said immunomodulatory polypeptide:
(a) activates antigen presenting cells;
(b) comprises a toll-like receptor modulator;
(c) comprises a type-4 toll-like receptor agonist; or
(d) galectin-1, galectin-3, LAG-3, or interleukin-10; and/or
(ii) to express a nucleic acid that encodes a polypeptide that targets the cell for phagocytosis; and/or
(iii) to express at least one growth-promoting protein, preferably wherein said growth-promoting protein is v-myc, N-myc, c-myc, p53, SV40 large T antigen, polyoma large T antigen, E1a adenovirus or E7 protein of human papillomavirus.

11. Modified erythrocytes produced by the mammalian precursor cell of any one of claims 1 to 10, optionally wherein the erythrocytes are:
(i) type O, type A, type B, or type AB; and/or
(ii) Rh negative or Rh positive; or
(iii) any of:
(a) blood type M, blood type N, blood type S, or blood type s;
(b) blood type P1;
(c) blood type Lua, blood type Lub, or blood type Lu(a);
(d) blood type K (Kell), k (cellano), Kpa, Kpb, K(a+), Kp(a-b-) or K-k-Kp(a-b-);
(e) blood type Le(a-b-), Le(a+b-) or Le(a-b+);
(f) blood type Fy a, Fy b or Fy(a-b-); or
(g) blood type Jk(a-b-), Jk(a+b-), Jk(a-b+) or Jk(a+b+).

12. The modified erythrocytes of claim 11, wherein said polypeptide that is not normally present on or in erythrocytes is a membrane-bound polypeptide or a soluble protein contained within the interior of said erythrocyte.

13. A modified erythrocyte produced by the mammalian erythrocyte precursor cell of claim 4 for use in a method of treating cancer in a subject in need thereof.

14. A modified erythrocyte produced by the mammalian erythrocyte precursor cell of claim 5 for use in a method of treating a viral disease or infection in a subject in need thereof.

15. A modified erythrocyte produced by the mammalian erythrocyte precursor cell of claim 6 for use in a method of treating a bacterial disease or infection in a subject in need thereof.

16. A modified erythrocyte produced by the mammalian erythrocyte precursor cell of claim 7 for use in a method of inducing tolerance to a self antigen in a subject in need thereof.
